(19) Europäisches Patentamt
European Patent Office
Office européen des brevets

(11) Publication number: **0 092 771**
**B1**

(12) **EUROPEAN PATENT SPECIFICATION**

(45) Date of publication of patent specification: **23.07.86**

(51) Int. Cl.$^4$: **C 12 M 1/36**

(21) Application number: **83103784.1**

(22) Date of filing: **19.04.83**

(54) **Process and apparatus for culture of microorganisms using oxygen-enriched gas.**

(30) Priority: **23.04.82 JP 67430/82**

(43) Date of publication of application:
**02.11.83 Bulletin 83/44**

(45) Publication of the grant of the patent:
**23.07.86 Bulletin 86/30**

(84) Designated Contracting States:
**CH DE FR GB IT LI NL SE**

(56) References cited:
**EP-A-0 052 890**
**CH-A- 571 359**
**US-A-3 856 671**
**US-A-3 926 737**
**US-A-4 064 015**

**MICROBIOLOGY ABSTRACTS, no. 6, June 1974, page 93, column 1, abstract no. 9A 3652, GB**

(73) Proprietor: **HITACHI, LTD.**
**6, Kanda Surugadai 4-chome Chiyoda-ku**
**Tokyo 100 (JP)**

(72) Inventor: **Shimizu, Norio**
**2374-24, Okubocho**
**Hitachi-shi (JP)**
Inventor: **Ueno, Masao**
**3-4-505, Imafukuhigashi-2-chome**
**Johto-ku Osaka (JP)**
Inventor: **Odawara, Yoji**
**21-6, Nishinarusawacho-4-chome**
**Hitachi-shi (JP)**
Inventor: **Sumitani, Tomoaki**
**1166, Higashitoyoi**
**Kudamatsu-shi (JP)**
Inventor: **Fujimoto, Masakatsu**
**6-7-6, Nijigaoka**
**Hikari-shi (JP)**
Inventor: **Fujiwara, Kiyoshi**
**978-1, Hiratahigashi**
**Kudamatsu-shi (JP)**

(74) Representative: **Finck, Dieter et al**
**Patentanwälte v. Füner, Ebbinghaus, Finck**
**Mariahilfplatz 2 & 3**
**D-8000 München 90 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process and apparatus for culture of microorganisms using oxygen-enriched gas and in particular to an improved process and apparatus for culture of microorganisms with oxygen-enriched gas whereby microorganisms are aerobically cultured.

In the aerobic culture of microorganisms, a medium and microorganisms are stored in a fermenter in general. According to this type of process, the concentration of dissolved oxygen in the medium is controlled by providing a stirrer to stir the liquor and varying the speed in rpm's of the stirrer or the aeration rate.

However, in case of the culture of fungi mycelium of which is cut off under intense stirring, the stirrer speed cannot be raised above a given speed, but it is impossible to maintain the target concentration of dissolved oxygen merely by increasing the aeration rate. Moreover, the oxygen transfer rate attainable by the aeration is from 200 to 300 m mol/l/hour. Hence, in high cell concentration culture or the culture of microorganisms requiring a large amount of oxygen, it is impossible to maintain the concentration of dissolved oxygen at a level that does not inhibit the growth. For instance, in baker's yeast culture when the concentration of dissolved oxygen in the culture liquor falls to less than 0.2 ppm, i.e. anaerobic, the yeast produces ethanol, resulting in a considerably lower cell yield. It is generally known, moreover, that microorganisms produce carbon dioxide through respiration, and that this carbon dioxide has a growth-inhibiting effect on cells. Because this inhibition by carbon dioxide of cell growth is very pronounced in baker's yeast and inosin fermentation and the like, the concentration of dissolved carbon dioxide in the culture liquor must be kept low. No effective method has yet been developed, however, to prevent the growth inhibition by carbon dioxide.

The present invention aims at solving these problems. The object of the invention is to provide a process and apparatus for culture of microorganisms using oxygen-enriched gas that enable the yield of cell production to be raised, by controlling the concentrations of dissolved oxygen and dissolved carbon dioxide in the culture liquor to the respective target concentrations.

In order to achieve this object, a first aspect of the present invention relates to, in a process for cells wherein a culture liquid containing microorganisms is stored in a fermenter and the oxygen-enriched gas is supplied to the culture liquor, an improvement for controlling the dissolved oxygen and dissolved carbon dioxide concentrations of the culture liquor to the respective target concentrations by the control of the oxygen partial pressure of the oxygen-enriched gas prepared by the adsorption of nitrogen from air and the control of the carbon dioxide partial pressure of the gas phase in the fermenter. A second aspect of the present invention relates to an improvement for controlling the concentrations of dissolved oxygen and dissolved carbon dioxide in a culture liquor to the respective target concentrations by providing a fermenter for storing a culture liquor containing microorganisms, an oxygen-enriched gas production unit for preparing oxygen-enriched gas by the adsorption of nitrogen from air and supplying the oxygen-enriched gas to the culture liquor, a dissolved oxygen concentration detector for detecting the concentration of dissolved oxygen in the culture liquor and outputting an oxygen concentration signal, a carbon dioxide partial pressure detector for detecting the carbon dioxide partial pressure of the gas phase in the fermenter and outputting a carbon dioxide partial pressure signal, a carbon dioxide remover for adsorbing carbon dioxide from the gas phase in the fermenter and for returning the gas from which the carbon dioxide has been adsorbed back to the fermenter, and a control unit for outputting a signal to control either the pressure or gas retention time, or both the pressure and gas retention time, within said oxygen-enriched gas production unit based on said oxygen concentration signal and also for outputting a signal to control either the pressure or gas retention time, or both the pressure and gas retention time, within said carbon dioxide remover based on said carbon dioxide partial pressure signal.

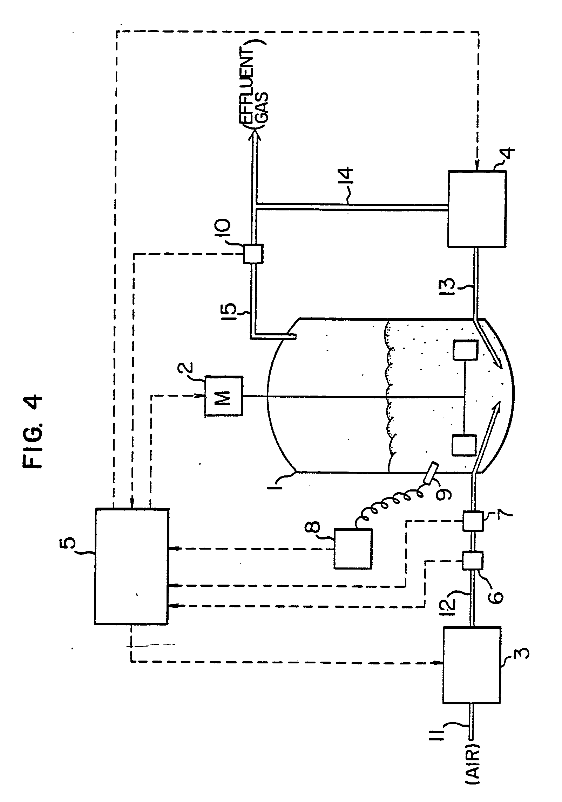

Fig. 1 shows the relationship between the stirring speed and the oxygen transfer rate at different oxygen partial pressures, Fig. 2 shows the relationship between the aeration rate and the oxygen transfer rate at a constant stirring speed, Fig. 3 shows the relationship between the gas flow rate and the outlet oxygen concentration, and Fig. 4 is a block diagram showing one embodiment of the second aspect of the present invention.

Figs. 1 and 2 show respectively the change in the oxygen transfer rate when the oxygen partial pressure of the oxygen-enriched gas supplied to the fermenter is varied, and when the aeration rate is varied. The oxygen transfer rate means a change with time in the amount of oxygen transferred from a gas phase to a liquid phase and is defined by the following equation:

Oxygen transfer rate = oxygen transfer rate coefficient $\times$ $(C^* - C)$ where $C^*$ is a saturated dissolved oxygen concentration and C is a dissolved oxygen concentration at that time.

The oxygen transfer rate coefficient is represented by $k_{LA}$ and is one of mass transfer rate coefficients [see Eckenfelder, W. W.: Adsorption of Oxygen from Air Bubbles in Water, J. Sanitary Eng. Division, Proc. A.S.C.E. *85* No. 5A-4 (1959)]. In the case of using a sodium sulfite method, C = O. Line A in Fig. 1 represents the oxygen transfer rate at an aeration rate of 1 VVM for air having an oxygen partial pressure of 0.21 atm, and line B represents the oxygen transfer rate for pure oxygen having an oxygen partial pressure of 1 atm. It is clear from Fig. 1 that the oxygen transfer rate rises about five-fold when the oxygen partial pressure of the aeration gas is raised from 0.21 atm for air to 1 atm for pure oxygen. It is furthermore apparent from Fig. 2, which shows the relationship between the aeration rate and oxygen transfer rate at a stirrer speed of 350 rpm, that doubling the aeration rate results in almost no increase in the oxygen transfer rate. This demonstrates that the oxygen transfer rate, or the concentration of dissolved oxygen in the culture liquor, can be controlled by controlling the oxygen partial pressure of the aeration gas. Fig. 1 shows that the concentration of dissolved oxygen can be controlled by controlling only the stirring speed, but this is not appropriate for the culture of fungi.

One possible method for controlling the partial pressure of oxygen is to connect an oxygen cylinder or an oxygen plant such as an electrolytic cell to the aeration gas line and control the rate of oxygen gas addition. However, because this requires both an air source and an oxygen plant, two separate piping and control valve systems are needed, in addition to which the amount of oxygen supplied is low, making this type of process inappropriate for large plants. Accordingly, in the present invention, it is preferable to control the partial pressure of oxygen with just an oxygen-enriched gas production unit such as an oxygen plant that works by pressure swing adsorption.

Oxygen plants that work by adsorption produce oxygen gas having a fixed concentration by adsorbing nitrogen gas onto an adsorbent. It can be seen from Fig. 3, which shows the relationship between the gas flow rate and outlet oxygen concentration in an oxygen plant, that the oxygen concentration, or partial pressure of oxygen, at the oxygen plant outlet can be controlled by varying the gas flow rate, or gas retention time. Moreover, the oxygen partial pressure of the outlet gas can be controlled by controlling the pressure within the oxygen plant. In other words, the oxygen partial pressure in the outlet gas can be increased by either raising the pressure in the oxygen plant or increasing the gas retention time within the oxygen plant.

And, by doing the reverse, the oxygen partial pressure of the outlet gas may be lowered.

According to the present invention, lowering the concentration of dissolved oxygen in the culture liquor raises the oxygen partial pressure of the oxygen-enriched gas, and raising the concentration of dissolved oxygen in the culture liquor lowers the oxygen partial pressure of the oxygen-enriched gas. In addition, enhanced control of the concentration of dissolved oxygen is possible by also controlling, for example, the stirring speed and the oxygen-enriched gas aeration rate.

Moreover, the present invention also provides for the control of the carbon dioxide partial pressure of the gas phase in the fermenter in order to prevent the inhibition of microorganism growth by dissolved carbon dioxide in the culture liquor. Because the concentration of dissolved carbon dioxide is in equilibrium with the carbon dioxide partial pressure of the gas phase in the fermenter, the concentration of dissolved carbon dioxide is controlled by controlling the carbon dioxide partial pressure of the gas phase in the fermenter. Thus, the carbon dioxide partial pressure in the fermenter is reduced through dilution by supplying exhaust gas from the top of the fermenter to the adsorption carbon dioxide remover, removing the carbon dioxide by adsorption, and returning the gas back to the fermenter at a lower carbon dioxide partial pressure.

The higher the oxygen partial pressure, the higher the carbon dioxide pressure is created. Particularly, in the process of culture using oxygen-enriched, gas, cell growth inhibition takes place very often. So, the carbon dioxide remover should be operated, when the partial pressure of carbon dioxide rises as a result of the accumulated consumption of a large amount of oxygen during the later half of the culture phase. The amount of oxygen supplied to the fermenter may increase as a result of the return of gas having a lowered carbon dioxide partial pressure to the fermenter. In such cases, the concentration of dissolved oxygen should be controlled by reducing the stirring speed. On the other hand, there are some species of microorganisms which have scarcely inhibition of growth by carbon dioxide. In such cases, although it is not essential to control the partial pressure of carbon dioxide, oxygen production costs may be lowered by raising oxygen utilization rate through the control of the carbon dioxide partial pressure. In addition, with regard to the control of the carbon dioxide partial pressure by the adsorption of carbon dioxide, the amount of carbon dioxide adsorbed in the adsorption unit can be controlled by controlling the pressure and gas retention time within the adsorption unit. Thus, by controlling the pressure and gas retention time of carbon dioxide in the adsorption, the partial pressure of carbon dioxide in the fermenter can be controlled to the target pressure.

Fig. 4 shows one embodiment of the second aspect of the invention. Fermenter 1 for storing the culture liquor comprising a medium and microorganisms contains the stirring element of a stirrer 2 provided with a motor M. An oxygen-enriched gas production unit 3 provided with an air inlet pipe 11 is connected to the bottom of 1 via oxygen partial pressure detector 6 and aeration gas rate detector 7. The top of the fermenter is connected with the carbon dioxide remover 4 via a pipe 15 provided with a carbon dioxide partial pressure detector 10 and an exhaust gas pipe 15. This carbon dioxide remover 4 is connected with the bottom of fermenter 1 via a pipe 13. A dissolved oxygen concentration sensor 9 is provided at one side of fermenter 1 such as to be in contact with the culture liquor, and to this dissolved oxygen concentration sensor 9 is attached an oxygen meter 8. Dissolved oxygen concentration sensor 9 and oxygen meter 8 together constitute the dissolved oxygen concentration detector.

Oxygen partial pressure detector 6, aeration gas rate detector 7, oxygen meter 8, and carbon dioxide partial pressure detector 10 are connected to a control unit 5 comprising a microcomputer and other components. Control unit 5 is connected to oxygen-enriched gas production unit 3, the motor M of stirrer 2, and carbon dioxide remover 4.

Adsorbent such as zeolite is provided in oxygen-enriched gas production unit 3 and carbon dioxide remover 4. When supplying gas to oxygen-enriched gas production unit 3 and carbon dioxide remover 4, it is desirable to install a moisture remover or the like and pretreat the gas by removing moisture from it. The dissolved oxygen concentration sensor 9 may be a polarographic or galvanometric device. An infrared spectrometer may be used as the carbon dioxide partial pressure detector 10.

The operation of the apparatus in this embodiment is described below. Air is introduced through air inlet pipe 11 into oxygen-enriched gas production unit 3, wherein the nitrogen is adsorbed by adsorbent from the air and the oxygen-enriched gas supplied to the culture liquor. As this is happening, the partial pressure of oxygen and the amount of exhaust gas are measured by oxygen partial pressure detector 6 and aeration gas rate detector 7, and input to control unit 5, which monitors these levels. Dissolved oxygen concentration sensor 9 detects the concentration of dissolved oxygen in the culture liquor and outputs an oxygen concentration signal via oxygen meter 8 to control circuit 5. The carbon dioxide partial pressure of the gas phase in fermenter 1 is measured by carbon dioxide partial pressure detector 10 and a carbon dioxide partial pressure signal output to control unit 5. Carbon dioxide is adsorbed onto the adsorbent in carbon dioxide remover 4, and the gas from which the carbon dioxide has been adsorbed is returned to the culture liquor via pipe 13. Based on the oxygen concentration signals and carbon dioxide partial pressure signals, control unit 5 outputs signals that control the pressure and gas retention time to oxygen-enriched gas production unit 3 and carbon dioxide remover 4, and controls the concentrations of dissolved oxygen and dissolved carbon dioxide in the culture liquor to the respective target concentrations. The pressures and gas retention times in oxygen-enriched gas production unit 3 and carbon dioxide remover 4 are controlled by the opening and closing of valves provided at the inlets and outlets of these units. For example, the gas retention time can be controlled by controlling the time at which the outlet valve is closed and the inlet valve opened to let in air, and the time at which the inlet valve is closed and the outlet valve opened to let out the oxygen-enriched gas. Alternatively, the pressure in the oxygen-enriched gas production unit can be controlled by connecting a compressor or the like to air inlet pipe 11 and controlling the compressor with the outlet valve closed and the inlet valve open.

Furthermore, the stirrer speed may be controlled should it be judged through computation by control unit 5 that the dissolved oxygen concentration can be more effectively controlled by increasing the stirring speed of stirrer 2 rather than by increasing or decreasing the oxygen partial pressure of the oxygen-enriched gas. The description of the apparatus given above applies for cases in which the oxygen-enriched gas production unit and the carbon dioxide remover are separately provided, but these two units may also be combined into a single adsorption unit. In such a combined unit, exhaust gas pipe 14 is connected to air inlet pipe 11.

As described above, according to this embodiment, oxygen-enriched gas at the target concentration can be supplied to the fermenter, and the accuracy of dissolved oxygen concentration control enhanced, by a single oxygen-enriched gas production unit. Moreover, because the carbon dioxide generated by microorganism respiration can be removed, the cell growth rate and yield can be increased. Another outstanding advantage is that the gas from which carbon dioxide has been removed is returned to the fermenter, enabling an increase in oxygen utilization rate.

As indicated above, the present invention provides the excellent effects of enabling the improved yield of product by controlling the concentrations of dissolved oxygen and dissolved carbon dioxide in the culture liquor and, because oxygen-enriched gas is produced from air, of being applicable to large plants.

## Claims

1. In a process for culture of microorganisms using an oxygen-enriched gas wherein a culture liquor containing microorganisms is stored in a fermenter and the microorganisms are cultured by supplying oxygen-enriched gas to the culture liquor, the improvement which comprises controlling the dissolved oxygen and dissolved carbon dioxide concentrations of the culture liquor to the respective target concentrations by controlling the oxygen partial pressure of the oxygen-enriched gas prepared by the adsorption of nitrogen from air and controlling the carbon dioxide partial pressure of the gas phase in said fermenter.

2. An apparatus for culture of microorganisms using oxygen-enriched gas, comprising a fermenter for storing a culture liquor containing microorganisms, an oxygen-enriched gas production unit for preparing oxygen-enriched gas by the adsorption of nitrogen from air and supplying said oxygen-enriched gas to said culture liquor, a dissolved oxygen concentration detector for detecting the concentration of dissolved oxygen present in said culture liquor and outputting an oxygen concentration signal, a carbon dioxide partial pressure detector for detecting the carbon dioxide partial pressure of the gas phase in said fermenter and outputting a carbon dioxide partial pressure signal, a carbon dioxide remover for adsorbing carbon dioxide from the gas phase in said fermenter and returning the gas from which said carbon dioxide has been adsorbed back to said fermenter, and a control unit for outputting a signal to control either the pressure or gas retention time, or both the pressure and gas retention time, within said oxygen-enriched gas production unit based on said oxygen concentration signal and also for outputting a signal to control either the pressure or gas retention time, or both the pressure and gas retention time, within said carbon dioxide remover based on said carbon dioxide partial pressure signal.

**Patentansprüche**

1. Verfahren zur Züchtung von Mikroorganismen unter Verwendung eines mit Sauerstoff angereicherten Gases, bei dem eine Mikroorganismen enthaltende Kulturflüssigkeit in einem Fermenter gehalten wird und die Mikroorganismen durch Zufuhr von mit Sauerstoff angereichertem Gas zur Kulturflüssigkeit gezüchtet werden, wobei die Verbesserung des Verfahrens darin besteht, daß die Konzentrationen an gelöstem Sauerstoff und gelöstem Kohlendioxid in der Kulturflüssigkeit auf die entsprechenden Zielkonzentrationen gebracht werden durch Regulieren des Sauerstoffpartialdrucks des durch Adsorption von Stickstoff aus der Luft hergestellten, mit Sauerstoff angereicherten Gases und durch Regulieren des Kohlendioxidpartialdrucks der Gasphase in dem Fermenter.

2. Vorrichtung zur Züchtung von Mikroorganismen unter Verwendung von mit Sauerstoff angereichertem Gas, wobei die Vorrichtung einen Fermenter für die Aufbewahrung einer Mikroorganismen enthaltenden Kulturflüssigkeit, eine Einrichtung zur Herstellung von mit Sauerstoff angereichertem Gas mittels Adsorption von Stickstoff aus der Luft und zur Zufuhr des mit Sauerstoff angereicherten Gases in die Kulturflüssigkeit, einen Detektor für den Nachweise der Konzentration des in der Kulturflüssigkeit vorhandenen gelösten Sauerstoffes und zum Signalisieren der Sauerstoffkonzentration, einen Detektor für den Nachweis des Kohlendioxidpartialdrucks der Gasphase in dem Fermenter und zum Signalisieren des Kohlendioxidpartialdrucks, einen Kohlendioxidausscheider zur Adsorption des Kohlendioxids aus der Gasphase im Fermenter und Rückführung des Gases, aus dem das Kohlendioxid adsorbiert worden ist, zum Fermenter, und einer Regler enthält zur Erzeugung eines Signals, um, ausgehend von dem Signal der Sauerstoffkonzentration, entweder den Druck oder die Gasretentionszeit, oder sowohl den Druck als auch die Gasretentionszeit in der Einrichtung zur Herstellung des mit Sauerstoff angereicherten Gases regulieren zu können, und außerdem zur Erzeugung eines Signals, um, ausgehend von dem Kohlendioxidpartialdrucksignal, entweder den Druck oder die Gasretentionszeit, oder sowohl den Druck als auch die Gasretentionszeit in dem Kohlendioxidausscheider regulieren zu können.

**Revendications**

1. Dans un processus pour réaliser la culture de micro-organismes moyennant l'utilisation d'un gaz enrichi en oxygène, selon lequel un bouillon de culture contenant des micro-organismes est stocké dans un dispositif de fermentation et les micro-organismes sont cultivés par envoi de gaz enrichi en oxygène au bouillon de culture, le perfectionnement consistant à régler les teneurs en oxygène dissous et en gaz carbonique dissous du bouillon de culture sur les teneurs de consigne respectives, par réglage de la pression partielle d'oxygène du gaz enrichi en oxygène, préparé par adsorption de l'azote à partir de l'air et par réglage de la pression partielle du gaz carbonique de la phase gazeuse dans ledit dispositif de fermentation.

2. Appareil pour la culture de micro-organismes moyennant l'utilisation de gaz enrichi en oxygène, comprenant un dispositif de fermentation servant à stocker un bouillon de culture contenant des micro-organismes, une unité de production d'un gaz enrichi en oxygène servant à préparer un gaz enrichi en oxygène au moyen de l'adsorption de l'azote à partir de l'air et pour envoyer ledit gaz enrichi en oxygène audit bouillon de culture, un détecteur de la teneur en oxygène dissous, servant à détecter la teneur en oxygène dissous présent dans ledit bouillon de culture et à délivrer un signal de teneur en oxygène, un détecteur de la pression partielle de gaz carbonique, servant à détecter la pression partielle de gaz carbonique de la phase gazeuse dans ledit dispositif de fermentation et à délivrer un signal de pression partielle de gaz carbonique, un dispositif d'élimination du gaz carbonique servant à adsorber le gaz carbonique à partir de la phase gazeuse, à l'intérieur dudit dispositif de fermentation, et à renvoyer le gaz, dont le gaz carbonique a été retiré par adsorption, audit dispositif de fermentation, et une unité de commande pour délivrer un signal servant à commander soit la pression, soit la durée de séjour du gaz, soit à la fois la pression et la durée de séjour du gaz, dans ladite unité de production d'un gaz enrichi en oxygène, sur la base dudit signal de la teneur en oxygène et également pour délivrer un signal servant à régler soit la pression, soit la durée de séjour du gaz, soit à la fois la pression et la durée de séjour du gaz à l'intérieur dudit dispositif d'élimination du gaz carbonique, sur la base dudit signal de pression partielle du gaz carbonique.

FIG. 1

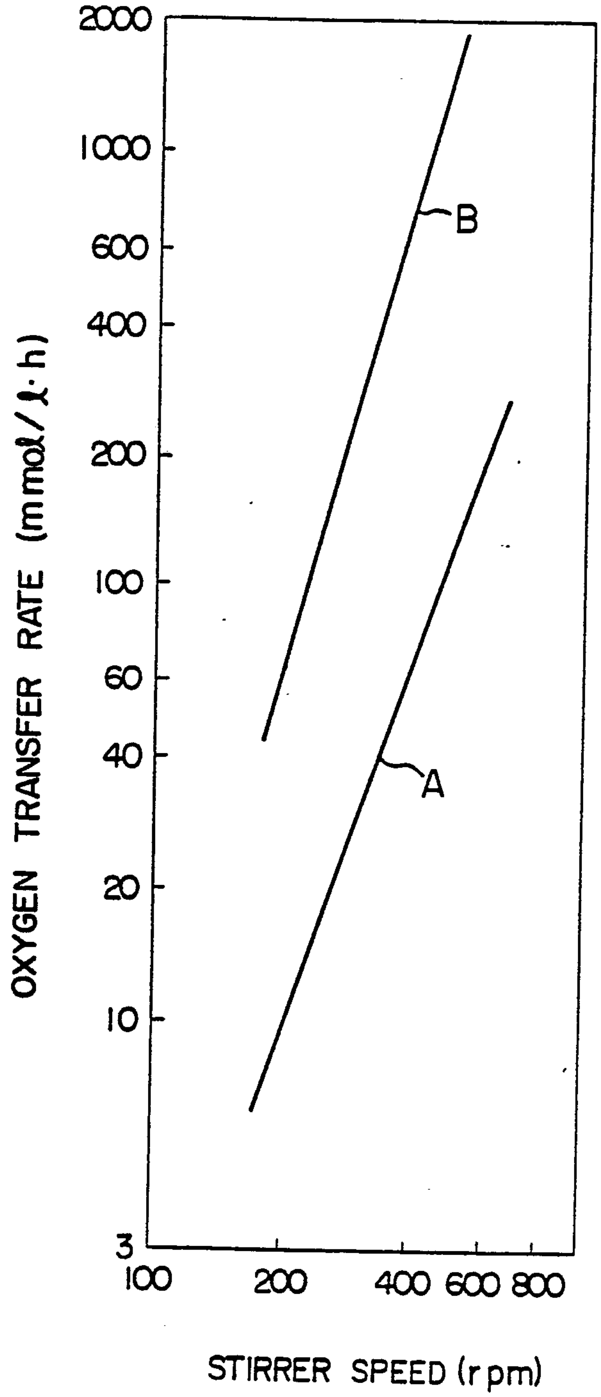
OXYGEN TRANSFER RATE (mmol/l·h)
2000
1000
600
400
200
100
60
40
20
10
3
100
200
400
600
800
STIRRER SPEED (rpm)
B
A

FIG. 2

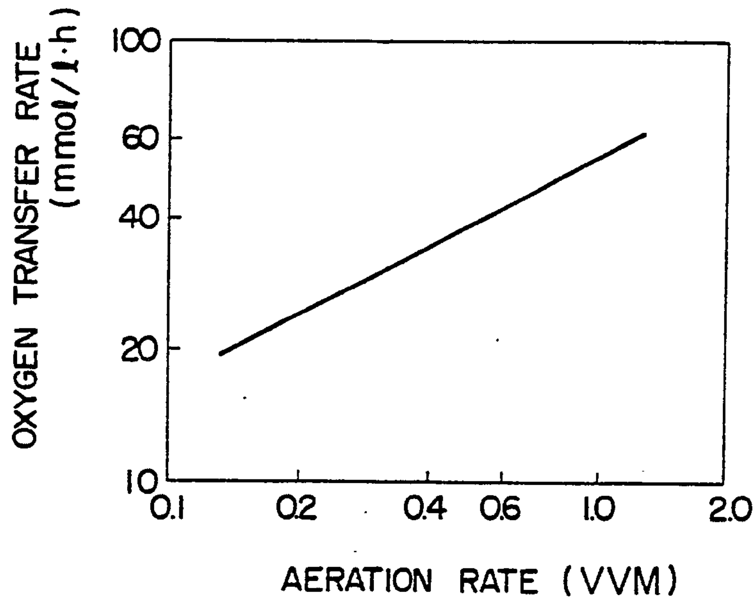
OXYGEN TRANSFER RATE (mmol/l·h)
100
60
40
20
10
0.1
0.2
0.4
0.6
1.0
2.0
AERATION RATE (VVM)

FIG. 3

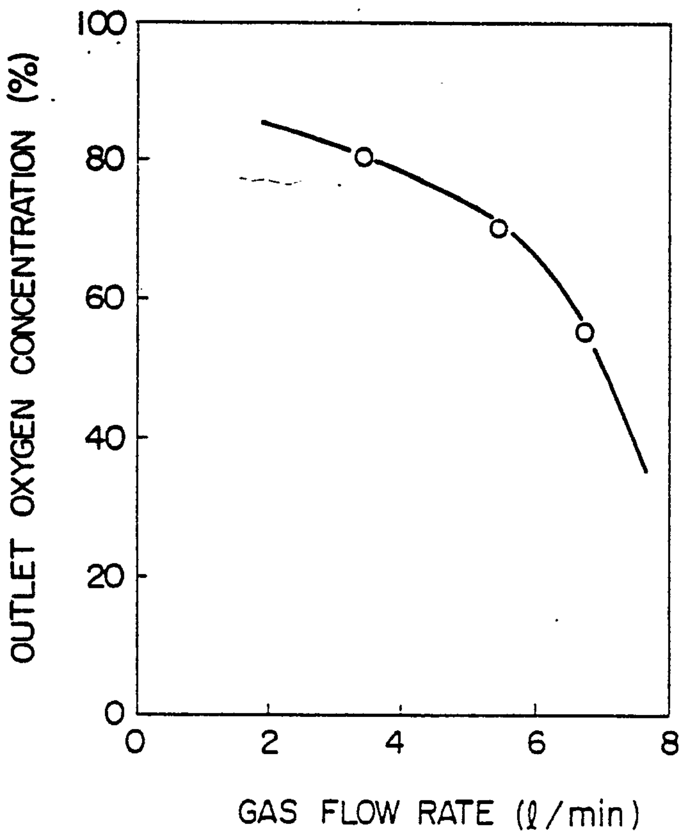
OUTLET OXYGEN CONCENTRATION (%)
100
80
60
40
20
0
0
2
4
6
8
GAS FLOW RATE (l/min)

FIG. 4